# EUROPÄISCHE PATENTANMELDUNG

(11) **EP 0 682 013 A1**
(43) Veröffentlichungstag der Anmeldung: **15.11.1995**
(21) Anmeldenummer: 95107124.0
(22) Anmeldetag: 11.05.1995
(51) Int. Cl.: C07D 207/08

(54) **Neues Verfahren zur Herstellung von enantiomerenreinen Diarylprolinolen**

(30) Priorität: 13.05.1994 DE 4416963
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Klinger, Franz Dietrich, Dr., D-64347 Griesheim (DE); Sobotta, Rainer, Dr., D-55218 Ingelheim am Rhein (DE)

(57) **Zusammenfassung**

Die vorliegende Erfindung betrifft ein neues Verfahren zur Herstellung von enantiomerenreinen Diarylprolinolen, insbesondere von (R)-(+) bzw. (S)-(-)-2-(Diphenylhydroxymethyl)-pyrrolidin (R-(+) bzw. S-(-)-α,α-Diphenyl-(2-pyrrodilinyl)-methanol) ausgehend von Prolin.

## Beschreibung

Die vorliegende Erfindung betrifft ein neues Verfahren zur Herstellung von enantiomerenreinen Diarylprolinolen, insbesondere von (R)-(+)- bzw. (S)-(-)-2-(Diphenylhydroxymethyl)-pyrrolidin ((R)-(+)- bzw. (S)-(-)-α,α-Diphenyl-(2-pyrrolidinyl)-methanol) ausgehend von Prolin.

Diarylprolinole - insbesondere Diphenylprolinole - und - namentlich - Di-2-naphthylprolinol [Tetrahedron Lett. 31, 601 (1990)] - finden als chirale Katalysatoren für die enantioselektive Reduktion von prochiralen Ketonen Verwendung und können auch bereits bei Reduktionen im industriellen Maßstab eingesetzt werden.

Dabei wird das eigentlich reduzierende Agenz durch die entsprechenden Oxazaboralidine verkörpert, die aus der Umsetzung der Diarylprolinole mit geeigneten Boranen hervorgehen.

Die Entdeckung der Möglichkeit, Oxazaborolidine bei der enantioselektiven Reduktion prochiraler Ketone einzusetzen, hat zu einem großen Interesse an dieser Verbindungsklasse geführt. [G. J. Quallich und T. M. Woodall, Synlett. 1993, 929].

Insbesondere in den letzten beiden Jahrzehnten sind einige Syntheserouten zur Herstellung dieser Verbindungen publiziert worden. Das zentrale Zwischenprodukt wird dabei durch die Diarylprolinole verkörpert. Erst der letzte Schritt beinhaltet - wie schon eingangs erwähnt - die Umsetzung mit dem gewünschten Boran.

So sind z.B. für (S)-1,1-Diphenylprolinol zahlreiche verschiedene Herstellungswege bekannt. Beispielsweise offenbart die französische Patentschrift 976.435 ein Verfahren, bei dem der Ethylester des 1-Prolins mit Phenylmagnesiumbromid zu dem entsprechenden α-Pyrrolidinyldiphenylcarbinol umgesetzt wird. Die mit diesem Verfahren erzielbare Produktausbeute ist aber eher als gering zu bezeichnen.

Daneben wird von D. Enders et al. ein Herstellverfahren beschrieben, bei dem Pyrrolidin mit Ethylnitrit zunächst in das entsprechende N-Nitrosamin überführt wird, welches nach der Deprotonierung mit Diisopropylamid und Umsetzung mit Benzophenon sowie anschließender reduktiver Abspaltung der Nitroso-Schutzgruppe das gewünschte 2-(Diphenylhydroxymethyl)pyrrolidin in Form einer racemischen Mischung liefert [D. Enders, R. Pieter, B. Renger und D. Seebach, Org. Syn. 58 (1978) 113]. Neben dem letztgenannten Nachteil (Racemat) wird in diesem Verfahren das N-Nitrosopyrrolidin als Zwischenstufe durchlaufen. Von N-Nitrosopyrrolidin ist jedoch bekannt, daß es im Tierversuch die Tumorbildung induziert.

Weitere Verfahren werden u.a. von Corey et al. [J. Am. Chem. Soc. 109 (1987) 7926], Kapfhammer et al. [Hoppe-Seylers Zeit. Physiol. Chem. 223 (1933) 43] und in der deutschen Offenlegungsschrift 3 609 152 beschrieben.

Daneben wurde von E. J. Corey eine mehrstufige Synthese beschrieben [J. Am. Chem. Soc. 109 (1987) 5551], bei der das (S)-1,1-Diphenylprolinol in einer Gesamtausbeute von 30 - 40% erhalten wird. Dieses Verfahren macht jedoch die Isolsierung sämtlicher Zwischenprodukte - wie N-(Benzyloxycarbonyl)-S-prolin und N-(Benzyloxycarbonyl)-S-prolinmethylester - erforderlich. Neben der Verwendung einer relativ teuren Schutzgruppe besitzt dieses Verfahren den Nachteil, daß zur Durchführung der Grignard-Reaktion des N-(Benzyloxycarbonyl)-S-prolinmethylesters ein Überschuß von sieben Equivalenten Phenylmagnesiumchlorid benötigt wird. Daneben bereitet die Isolierung des Diphenylprolinols - in Gegenwart der in großen Mengen aus der Grignard-Reaktion herrührenden Magnesiumverbindungen - technische Schwierigkeiten. Hierzu muß das Magnesiumhydroxidgel mehrfach extrahiert werden.

Eine einfachere Herstellmethode wird in der europäischen Patentanmeldung EP-A-O 453 298 offenbart. Ausgehend von S-Prolin wird zunächst das (S)-Tetrahydro[1H,3H]pyrrolo-1,2-c]oxazol-1,3-dion hergestellt; dazu muß S-Prolin zunächst mit Phosgen zur Reaktion gebracht und im nachfolgenden Reaktionsschritt mit dem Triethylamin umgesetzt werden [Fuller et al., Biopolymers 15, (1976) 1869] (vgl. Beispiel 1). Das resultierende Carboxanhydrid wird mit dem entsprechenden Arylmagnesiumhalogenid - hier Phenylmagnesiumchlorid - zum gewünschten (S)-α,α-Diphenyl-2-pyrrolidinmethanol umgesetzt, welches auf diesem Wege in einer Ausbeute von 73 % d. Th. zugänglich ist. Der Nachteil dieses Verfahrens besteht jedoch darin, daß - bedingt durch die Verwendung von Phosgen - erhöhte sicherheitstechnische Anforderungen an die Reaktionsapparatur gestellt werden müssen.

Unter dem Gesichtspunkt einer möglichst kostengünstigen und sicheren Herstellung im industriellen Maßstab sind die aus dem Stand der Technik bekannten Verfahren nicht geeignet.

Es ist daher die Aufgabe der vorliegenden Erfindung, die Nachteile der aus dem Stand der Technik bekannten Herstellverfahren zu überwinden.

Erfindungsgemäß wird diese Aufgabe dadurch gelöst, daß im Prolin in der ersten Reaktionsstufe mit Benzylchlorid zum einen der Pyrrolidin-Stickstoff mit der entsprechenden Benzyl-Schutzgruppe geschützt und gleichzeitig die freie Carboxylfunktion in den korrespondierenden Benzylester überführt wird.

In der zweiten Reaktionsstufe wird durch Umsetzung mit einem Arylmagnesiumhalogenid - beispielsweise durch Umsetzung mit Phenylmagnesiumchlorid - das entsprechende Grignard-Produkt - in diesem Fall das N-Benzyl-2-(diphenylhydroxymethyl)-pyrrolidin - erhalten.

Auf der letzten Reaktionsstufe wird die Benzylschutzgruppe auf dem Weg der katalytischen Hydrierung abgespalten.

Neben dem Vorzug, daß bei der erfindungsgemäß vorgeschlagenen Reaktionsfolge keine sicherheitsbedenklichen Agenzien eingesetzt werden, weist das erfindungsgemäße Verfahren den weiteren Vorteil auf, daß die Reaktionsprodukte, die nach den einzelnen Teilreaktionen erhalten werden, nicht isoliert bzw. gereinigt werden brauchen.

Die einzelnen Verfahrensschritte werden wie folgt durchgeführt:
Zur Durchführung der ersten Reaktionsstufe wird die gewünschte Menge D- oder L-Prolin in einem inerten Lösungsmittel vorgelegt. Als Reaktionsmedien zur Durchführung des Schutzes eignen sich alle Lösungsmittel, die aus dem Stand der Technik - insbesondere für Benzylierungsreaktionen - bekannt sind [vgl. T.W. Greene, Protektive Groups in Organic Synthesis, John Wiley & Sons, New York 1981]. Vorzugsweise werden organische Sulfoxide - wie Dimethylsulfoxid - oder Säureamide eingesetzt, worunter Dimethylformamid besonders bevorzugt wird. Der Schutz der Carboxylfunktion und der Aminofunktion - beispielsweise mit Aralkylhalogeniden - erfolgt vorzugsweise in Gegenwart basisch reagierender Verbindungen - wie z.B. geeigneter Alkali- oder Erdalkaliverbindungen - worunter Alkalisalze bevorzugt werden. Besonders bevorzugt wird Natriumhydrogencarbonat eingesetzt. Die für den gewünschten Schutz einzusetzenden Schutzgruppen sind ebenfalls aus dem Stand der Technik bekannt [s. T.W. Greene a.a.O.]. Zu diesem Zweck werden bevorzugt Alalkylhalogenide eingesetzt - besonders bevorzugt wird Benzylchlorid als Agenz eingesetzt. Die Reaktanden werden vorzugsweise bei erhöhter Temperatur umgesetzt. Bevorzugt erfolgt die Umsetzung in einem Temperaturintervall von 60 bis 140°C, wobei Temperaturen in einem Bereich von 80 bis 100°C besonders bevorzugt werden (wobei sich von selbst versteht, daß die Wahl der geeigneten Reaktionstemperatur von der Art bzw. Reaktivität der eingesetzten Reaktanden abhängig ist).

Nach erfolgter Reaktion wird das Reaktionsgemisch vorzugsweise auf eine Temperatur im Bereich von ca. 20 bis 40°C abgekühlt und mit Wasser versetzt.

Das so erhaltene Reaktionsgemisch wird anschließend mit einem geeigneten Extraktionsmittel extrahiert. Zu diesem Zweck eignen sich Kohlenwasserstoffe insbesondere Alkane - wie z.B - Petroletherfraktionen - oder aromatische Kohlenwasserstoffe, worunter Toluol als Extraktionsmittel besonders bevorzugt wird.

Nach dem Einengen der vereinigten Extrakte - vorzugsweise unter milden Temperaturen zweckmäßigerweise im Bereich von ca. 30 - 100°C - unter vermindertem Druck wird der verbliebene Destillationsrückstand ohne weitere Reinigungsoperationen in der zweiten Reaktionsstufe eingesetzt.

Im Rahmen des folgenden Syntheseschritts wird das zuvor geschützte Prolinderivat (beim Einsatz von Benzylhalogeniden als Agenz das entsprechende Dibenzylprolin) mit einer entsprechend ausgewählten Grignardverbindung zu dem gewünschten Prolinolderivat umgesetzt. Entsprechende Umsetzungen bzw. geeignete Grignardverbindungen sind - an sich - aus dem Stand der Technik bekannt. [J. March, Advanced Organic Chemistry, 3^{rd} Edition, S. 434, John Wiley and Sons, New York, 1985 und zit. Lit.].

Vorzugsweise finden Arylmagensiumhalogenide als Grignardverbindungen Verwendung. So wird z.B. zur Herstellung von Diphenylprolinol besonders bevorzugt Phenylmagnesiumchlorid eingesetzt. - Als weitere Grignardverbindungen seien β-Naphthylmagnesiumhalogenide genannt.

Zur Durchführung der Grignardreaktion wird - beispielsweise - das in der ersten Stufe geschützte Prolinderivat in Lösung mit dem Grignardreagenz zur Reaktion gebracht. Als Lösungsmittel eignen sich alle unter den gegebenen Reaktionsbedingungen inerten Lösungsmittel, die den Reaktionsverlauf nicht nachteilig beeinflussen. Die hierfür in Frage kommenden Lösungsmittel sind aus dem Stand der Technik bekannt. Vorzugsweise werden Ether eingesetzt, worunter cyclische Ether - wie zum Beispiel Tetrahydrofuran - besonders bevorzugt werden. Die Umsetzung wird - in Abhängigkeit von der Reaktivität der Edukte - bei erhöhter Temperatur durchgeführt. Bevorzugt wird eine Reakionstemperatur im Bereich von 50 bis 90°C, besonders bevorzugt wird eine Reaktionstemperatur bei 70°C. Die Umsetzung wird ferner vorzugsweise unter Inertgasatmosphäre durchgeführt.

Nach erfolgter Umsetzung wird die Reaktionsmischung nach dem Abkühlen auf Raumtempratur - d.h. eine Temperatur im Bereich von 15 bis 35°C - hydrolisiert. Die Hydrolyse wird dabei vorzugsweise mit der wässerigen Lösung einer Säure durchgeführt, wobei die verdünnte wässerige Lösung einer Mineralsäure - wie z.B. Schwefelsäure - besonders bevorzugt wird. In Abhängigkeit von der Exothermie der Hydrolysereaktion kann es erforderlich sein, die Reaktionsmischung während der Hydrolyse zu kühlen.

Nach vollendeter Hydrolyse wird ggf. mittels weiterer Säurezugabe ein pH-Wert im Neutralbereich - bevorzugt von 7 - eingestellt. Die wässerige Phase wird nochmals mit einem organischen Lösungsmittel - vorzugsweise dem ursprünglich gewählten Ether und besonders bevorzugt mit Tetrahydrofuran - ausgerührt. Die organische Phase wird ggf. bei erhöhter Temperatur, die vorzugsweise in einem Intervall von 50 bis 60°C liegt, im Wasserstrahlvakuum eingeengt und der Rückstand - ohne weitere Reinigungsschritte - isoliert.

In der letzten Stufe wird die Schutzgruppe von der Aminofunktion abgespalten. Die Verfahren zum Entfernen der Schutzgruppen sind - an sich - ebenfalls aus dem Stand der Technik bekannt. So kann z.B. eine Benzylschutzgruppe unter reduktiven Bedingungen leicht abgespalten werden. Dazu wird beispielsweise das entsprechende N-Benzylprolinolderivat in einem geeigneten Lösungsmittel - vorzugsweise einem Alkohol und besonders bevorzugt in Ethanol - gelöst und in Gegenwart eines Katalysators - vorzugsweise Palladium auf Kohle, wobei ein Katalystor mit einem Palladiumgehalt von 10 % besonders bevorzugt wird - mit Wasserstoff unter erhöhtem Druck - vorzugsweise in einem Druckbereich von 2 bis 20 bar, besonders bevorzugt von 3 - 7 bar - umgesetzt. Nach vollendeter Hydrogenolyse der N-Benzyl-Bindung wird der Katalysator abfiltriert und das Filtrat unter vermindertem Druck - vorzugsweise im Wasserstrahlvakuum - und in Abhängigkeit vom eingesetzten Reaktionsmedium - bei erhöhter Temperatur bis 70°C eingeengt. Zur Reinigung bzw. Isolierung des,so hergestellten Prolinolderivats wird der verbliebene Rückstand in einem geeigneten Lösungsmittel - vorzugsweise in einem aliphatischen oder aromatischen Kohlenwasserstoff, besonders bevorzugt in Toluol - gelöst und filtriert. Das Filtrat wird mit der wässerigen Lösung einer Säure - vorzugsweise mit einer Mineralsäure, besonders bevorzugt mit 15-prozentiger Schwefelsäure - versetzt. In der wässerigen Lösung wird ein pH-Wert kleiner als 7, vorzugsweise ein pH-Wert im Bereich von 1 bis 4 und besonders bevorzugt ein pH-Wert im Bereich von 2 bis 3 eingestellt, wobei - nach turbulentem Rühren - das entsprechende Salz des eingesetzten α,α-Diarylprolinols ausfällt. Die resultierende Suspension wird abgenutscht und der Filterkuchen anschließend gewaschen.

Zur Freisetzung der Base wird das - ggf. noch nutschenfeuchte - Salz des Diarylprolinolderivats unter kräftigem Rühren in einem inerten Lösungsmittel, vorzugweise in einem halogenierten Kohlenwasserstoffund besonders bevorzugt in Dichlormethan aufgeschlämmt. Die Aufschlämmung wird anschließend - bis Entmischung eintritt - mit Wasser und dann mit der Lösung einer Base - vorzugsweise mit der verdünnten wässerigen Lösung einer Alkali- oder Erdalkaliverbindung und besonders bevorzugt mit 25 prozentiger wässeriger Natronlauge versetzt bis ein pH-Wert größer als 7 - bevorzugt im Bereich von 8 bis 12 und besonders bevorzugt ein pH-Wert von 9 - eingestellt ist. Nach dem intensiven Vermischen der organischen und der wässerigen Lösung sowie anschließender Phasentrennung sowie erschöpfender Extraktion der wässerigen Phase werden die vereinigten organischen Extrakte - vorzugsweise unter vermindertem Druck und besonders bevorzugt im Wasserstrahlvakuum eingeengt und das gewünschte Prolinolderivat isoliert.

Die eingangs genannten Aufgaben werden durch die in den Beispielen beschriebenen Verfahren gelöst. Veschiedenartige, andere Ausgestaltungen des Verfahrens werden für den Fachmann aus der vorliegendenden Beschreibung ersichtlich. Es wird jedoch ausdrücklich darauf hingewiesen, daß die Beispiele und die diesen zugeordnete Beschreibung lediglich zum Zweck der Erläutetung und Beschreibung vorgesehen und nicht als Einschränküng der Erfindung anzusehen sind.

### Beispiel 1 (Benzylierung):

In einem 6 l-Sulfierkolben werden 230 g (2 mol) L-Prolin in 2,4 l Dimethylformamid vorgelegt und mit 420 g (5 mol) Natriumhydrogencarbonat sowie mit 633 g (5mol) Benzylchlorid versetzt. Anschließend wird die Reaktionsmischung über einen Zeitraum von ca. 4 Stunden bei einer Temperatur von 100°C gerührt. Danach wird die resultierende Suspension auf Raumtemperatur abgekühlt und unter Rühren in 8.7 l Wasser eingetragen. Die wässerige Lösung wird dreimal mit Toluol extrahiert und die vereinigten Extrakte (ca. 1.3 l) im Vakuum bei 40 bis 50 Torr abdestilliert.

Bei einer Temperatur von ca. 80°C und unter einem Druck von 25-30 Torr werden Dimethylformamid und unumgesetztes Benzylchlorid destillativ abgetrennt, wonach 667 g Destillationsrückstand als braunes Öl verbleiben.
Die weitere Destillation über einen Zeitraum von 3 Stunden und bei einer Temperatur im Bereich von 80 - 120°C und einem Druck von 2 bis 10 Torr liefert 639 g eines braunen Öles (N-Benzyl-L-prolinbenzylester).

### Beispiel 2 (Grignard-Reaktion):

In einem mit Inertgas gespülten 4.5 l Kolben werden 657.5 ml (1.25 mol) einer 25 Gew.-proz. Lösung von Phenylmagnesiumchlorid vorgelegt und auf Rückflußtemperatur erhitzt. Innerhalb eines Zeitintervalls von 30 Minuten wird dann eine Lösung von 147.7 g (0.5 mol) N-Benzyl-L-prolinbenzylester in 500 ml Tetrahydrofuran unter Rühren zugetropft. Anschließend wird die Reaktionsmischung weiter auf Rückflußtemperatur erhitzt, bis dünnschichtchromatographisch praktisch kein N-Benzyl-L-prolinbenzylester mehr nachweisbar ist. Danach wird der Ansatz auf Raumtemperatur abgekühlt und die Reaktionslösung bei einer Temperatur im Bereich von 10 bis 20°C unter kräftigem Rühren mit 1500 ml verdünnter Schwefelsäure (300 ml 15 Gew.-proz. Schwefelsäure + 1200 ml Wasser) versetzt. Mit weiteren 26 ml 15 Gew.-proz. Schwefelsäure wird die Hydrolysemischung dann neutralisiert (pH=7). Die wässerige Phase wird mit Tetrahydrofuran ausgerührt. Nach erfolgter Phasentrennung und Extraktion werden die vereinigten Extrakte unter Wasserstrahlvakuum bei 55°C eingeengt, wonach 221.6 g des N-Benzyl-α,α-diphenylprolinols als Destillationsrückstand verbleiben.

### Beispiel 3 (Debenzylierung):

110.25 g des in Stufe 2 hergestellten N-Benzyl-α,α-diphenylprolinols werden in 1700 ml Ethanol gelöst und in Gegenwart von 18 g Katalysator - Palladium auf Kohle, 10 proz - bei 70°C unter einem Wasserstoffdruch von 5 bar über einen Zeitraum von ca. 5.5 Stunden hydriert. Danach wird der Katalysator abfiltriert. Das Filtrat wird unter Wasserstrahlvakuum bei einer Temperatur von ca. 60°C eingeengt, das α,α-Diphenyl-L-prolinol als, wonach Destillationsrückstand verbleibt (ca. 167 g).

### Beispiel 4 (Reinigung):

Der in Beispiel 3 erhaltene Destillationsrückstand wird in 2.5 l warmen Toluol gelöst und die resultierende - trübe - Lösung über ein Seitzfilter (0=10 cm) Supra 500 filtriert. Der Filterrückstand wird mit 500 ml Toluol gewaschen und das Filtrat wird mit weiteren 500 ml Toluol verdünnt. Unter kräftigem Rühren werden 142 ml 15 Gew.-proz. Schwefelsäure zugefügt, wobei sich in der Emulsion ein pH-Wert im Bereich von 2 - 3 einstellt und das Sulfat des α,α-Diphenyl-L-prolinols ausfällt. Die resultierende dickflüssige Suspension wird noch ca. 1 Stunde lang gerüht und über einen Zeitraum von ca. 12 Stunden bei Raumtemperatur zum Auskristallisieren stehengelassen. Danach wird die Suspension abgesaugt und der Nutschenkuchen mit 700 ml Toluol gewaschen. Das noch nutschenfeuchte Sulfat wird anschließend unter kräftigem Rühren in 3 l Dichlormethan aufgeschlämmt. Die Aufschlämmung wird mit 1 l Wasser sowie mit 53 ml 25 proz. Natronlauge versetzt, wobei sich ein pH-Wert von ca. 9 einstellt. Nach einstündigem Rühren werden die Phasen getrennt und die wässerige Phase mit jeweils 500 und 300 ml Dichlormethan erneut extrahiert. Die vereinigten organischen Extrakte werden im Wasserstrahlvakuum eingeengt und bei 50°C i. Vak. getrocknet, wonach 96.6 g (76.2 % d. Th.) des Diphenyl-L-prolinols in Form brauner Kristalle verbleiben.

## Patentansprüche

1. Verfahren zur Herstellung von enantiomerenreinen Diarylprolinolen, dadurch gekennzeichnet, daß man
a) D-oder L-Prolin in einem inerten Lösungsmittel - vorzugsweise in einem organischen Sulfoxid oder Säureamid - löst und gegebenenfalls in Gegenwart einer Verbindung eines Alkali- oder Erdalkalimetalls bei einer erhöhtem Temperatur - vorzugsweise im Bereich von 60 bis 140°C - mit einem Aralkylhalogenid umsetzt und nach dem Abkühlen mit Wasser vesetzt, das Reaktionsgemisch mit einem Koldenwasserstoff extrahiert und isoliert und
b) das geschützte D- bzw. L-Prolinderivat unter Inertgasatmosphäre in einem inerten Lösungsmittel - vorzugsweise in einem Ether - löst und bei erhöhter Temperatur - vorzugsweise im Bereich von 50 bis 90°C - mit einer Aryl-Grignardverbindung umsetzt und nach dem Abkühlen der Reaktionsmischung mit der verdünnten Lösung einer Säure - vorzugweise einer Mineralsäure - gegebenenalls unter Kühlen versetzt und einem pH-Wert im Neutralbereich einstellt und die wässerige Phase mit einem organischen Extraktionsmittel - vorzugsweise einem Ether - extrahiert und das Diarylprolinolderivat isoliert und
c) das N-Aralkylprolinderivat in einem Alkanol löst und in Gegenwart einer die reduktive Abspaltung der Aralkyl-Schutzgruppe katalysierenden Substanz mit Wasserstoff unter erhöhtem Druck - vorzugsweise im Bereich von 2 bis 20 - bar umsetzt und das von der Schutzgruppe befreite Prolinolderivat nach Entfernen des Reaktionsmediums in einem aliphatischen oder aromatischen Kohlenwasserstoff löst, die Lösung filtriert und das Filtrat mit der wässerigen Lösung einer Säure - vorzugsweise einer Mineralsäure - versetzt, bis ein pH-Wert von weniger als 7 erreicht ist, das aus dieser Umsetzung resultierende Salz isoliert und
d) dieses Salz in einem inerten Lösungsmittel - vorzugsweise in einem halogenierten Kohlenwasserstoff- aufschlämmt und mit Wasser bis zur Phasentrennung versetzt und anschließend mit der Lösung einer Base - vorzugsweise mit der wässerigen Lösung einer Alkali- oder Erdalkaliverbindung - einen pH-Wert im basischen Bereich - vorzugsweise im Berich von 8 bis 12 - einstellt und das aus seinem Salz freigesetzte Prolinolderivat extrahiert und isoliert.

2. Verfahren zur Herstellung von enantiomerenreinen Diarylprolinolen, dadurch gekennzeichnet, daß man
a) D- oder L-Prolin in Dimethylformamid löst und in Gegenwart eines Alkalisalzes bei einer erhöhten Temperatur vorzugsweise im Bereich von 60 bis 100°C mit Benzylchlorid umsetzt und nach dem Abkühlen auf eine Temperatur im Bereich von 20 bis 40°C mit Wasser versetzt, das Reaktionsgemisch mit Toluol extrahiert, das Reaktionsprodukt anschließend isoliert und
b) das geschützte D- bzw. L-Prolinderivat unter Inertgasatmosphäre in Tetrahydrofuran löst und bei einer Temperatur von 70°C mit einem Phenylmagnesium- oder β-Naphthylmagnesiumhalogenid umsetzt und nach dem Abkühlen der Reaktionsmischung auf eine Temperatur im Bereich von 15 bis 35°C mit verdünnter Schwefelsäure gegebenenfalls unter Kühlen versetzt und einem pH-Wert von 7 einstellt und die wässerige Phase mit Tetrahydrofuran extrahiert und das Diarylprolinolderivat isoliert und
c) das N-Aralkylprolinderiat in Ethanol löst und in Gegenwart Palladium auf Kohle (10 %ig) mit Wasserstoffunter einem Druck im Bereich von 3 bis 7 bar umsetzt und das von der Schutzgruppe befreite Prolinolderivat nach Entfernen des Reaktionsmediums in Toluol löst, die Lösung filtriert und das Filtrat mit 15 %iger Schwefelsäure versetzt bis ein pH-Wert im Bereich von 2 bis 3 erreicht ist, das aus dieser Umsetzung resultierende Salz isoliert und
d) das Salz in Dichlormethan aufschlämmt und mit Wasser bis zur Phasentrennung versetzt und anschließend mit 25 %-iger Natronlauge einem pH-Wert von 9 einstellt und das aus seinem Salz freigesetzte Prolinolderivat mit Dichlormethan extrahiert und isoliert.
